# EUROPEAN PATENT SPECIFICATION

(11) **EP 3 079 751 B1**
(45) Date of publication and mention of the grant of the patent: **17.04.2019**
(21) Application number: 14824256.3
(22) Date of filing: 10.12.2014
(51) Int. Cl.: A61M 25/00, A61L 29/04, A61L 29/08, A61L 29/14

(54) **WATER DISINTEGRABLE FLUSHABLE CATHETER WITH A HYDROPHILIC COATING**
IM WASSER ZERFALLENDER SPÜLBARER KATHETER MIT HYDROPHILER BESCHICHTUNG
CATHÉTER À JETER DANS LES TOILETTES QUI SE DÉSINTÈGRE DANS L'EAU, DOTÉ D'UN REVÊTEMENT HYDROPHILE

(30) Priority: 12.12.2013 US 201361915396 P; 12.12.2013 US 201361915370 P; 12.06.2014 US 201462011410 P
(43) Date of publication of application: 19.10.2016
(73) Proprietor: Hollister Incorporated, Libertyville, IL 60048 (US)
(72) Inventor: CLARKE, John, T., Galway (IE); MONTES DE OCA BALDERAS, Horacio, Ballina (IE); ROSTAMI, Shamsedin, Little Gransden South Cambridgeshire SG19 3DP (GB)
(74) Representative: FRKelly
(86) International application number: PCT/US2014/069534
(87) International publication number: WO 2015/089181

(56) References cited:
- EP-A1- 2 301 595
- EP-A2- 1 110 561
- WO-A1-96/41653
- WO-A1-2012/163413
- WO-A1-2014/193402

## Description

### Cross-reference to Related Applications

This application claims the benefit of and priority to U.S. Application Serial No. 61/915,370, filed December 12, 2013, and U.S. Application Serial No. 62/011,410, filed June 12, 2014, and U.S. Application Serial No. 61/915,396, filed December 12, 2013.

### Field of the Disclosure

The present disclosure is directed to urinary catheter products and, more particularly, to flushable catheters which include a surface treatment or coating that reduces friction to allow for easier and less traumatic insertion into and through the user's urethra.

### Background

Flushable urinary catheter products are desirable. However, to date only oil-based coatings have proved feasible for use with flushable catheters because water-based coatings dissolve or degrade these water-soluble, flushable catheters. Flushable catheters with oil-based coatings have lubricity values that, while better than gel products, may not always be as high as the lubricity values for hydrophilically coated catheters. This could be a problem for customers who desire flushable catheters with hydrophilic type lubricity values. An example of a degradable urinary catheter can be found in EP-A-2301595.

### Summary

The present disclosure provides flushable catheters that include hydrophilic outer coatings, as described in the claims. The catheters disclosed herein are catheters that structurally break down when contacted by water for convenient disposal down a toilet and through the sewer system. The catheters disclosed herein may be made from one or more materials that are affected by a fluid (for example, water, urine or fluids utilized in toilet and plumbing systems). Such materials may be water disintegratable or disintegrable materials. As used herein "water disintegratable" or "water disintegrable" materials refer to materials that are water soluble, water degradable, or water hydrolysable, and which dissolve, degrade, or otherwise break down when in contact with water over a selected period of time. In other examples, the material may be enzymatically hydrolysable. The water disintegratable and enzymatically hydrolysable materials are preferably flushable materials which are suitable for disposal in a toilet or sanitary system and, even more preferably, biodegradable flushable materials which may be chemically broken down by living organisms or other biological means.

In one embodiment, the water disintegrable, flushable catheter has a catheter shaft that is made from a bi-layer tube that has a biodegradable outer layer (such as polylactic acid or polylactide, PLA) that will support the hydrophilic coating even when it's wetted with, for example, water or an aqueous solution. The outer layer bonds well to the water disintegrable inner layer but also allows a hydrophilic coating to be applied on top of the outer layer. The biodegradable outer layer also serves as a barrier that prevents or slows water from contacting the water disintegrable inner layer of the catheter shaft prior to and during use. The coating can be activated at point of use or just before it.

In another example, the water disintegrable flushable catheter includes a catheter shaft formed from a water disintegrable material wherein the hydrophilic coating is applied directly to the outer surface of the catheter shaft. The hydrophilic coating is active or wetted with a non-aqueous activation agent or a mixture of an activation agent and water. In this case the coating can be preactivated during manufacturing or packaging.

This is a water disintegrable, flushable catheter with a biodegradable outer layer that degrades via hydrolysis and/or dissolution and a purely water disintegrable inner layer, e.g., polyvinyl alcohol (PVOH). The outer layer breaks down at a slower rate than the inner layer and bonds well to the PVOH inner layer. The outer layer acts as a stable primer layer for hydrophilic style coatings. The outer layer acts as a temporary water barrier. This water barrier allows the hydrophilic coating to be hydrated but prevents water molecules attacking the PVOH substrate and causing the hydrophilic coating to fall off. The outer layer has been shown to be compatible with hydrophilic coatings. In one embodiment, the hydrophilic coating may be wetted just prior to use by the user.

In another aspect not being part of the invention, a flushable catheter includes a catheter shaft that is made from a water disintegrable material, such as PVOH, wherein the water disintegrable catheter is coated with a hydrophilic coating. The hydrophilic coating is wetted or activated with a non-aqueous wetting/activation agent, such as propylene glycol (PG), polyethylene glycol (PEG), tetra-ethylene glycol, glycerol and tri-ethylene glycol. In one alternative embodiment, the wetting agent includes a mixture of a non-water based composition, such as any of those mentioned above, and an amount of water. The water may be in an amount between about 0 wt% to about 20 wt%, between about 0 wt% to about 10 wt% or between about 0 wt% to about 5 wt%.

### Brief Description of the Drawing

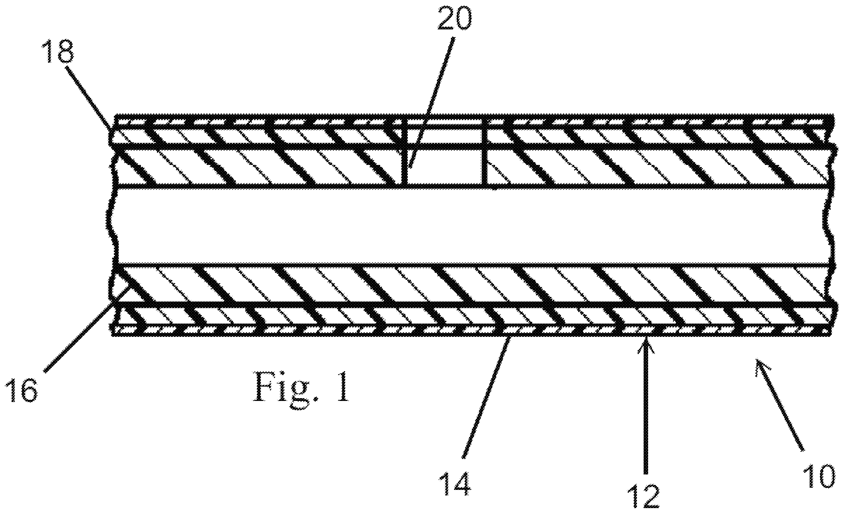

Fig. 1 is a longitudinal cross-section of a portion of a catheter having a bi-layer tube according to the present disclosure.

### Detailed Description of the Embodiments

The present disclosure is directed to a water disintegrable flushable catheter that will dissolve, hydrolyze or biodegrade in water. It is coated with a hydrophilic coating and will achieve lubricity values typical of a hydrophilic catheter. In the past hydrophilic coatings have been problematic for water disintegrable catheters because they typically need water to hydrate the coating. While the coating will become lubricious on hydration, the substrate catheter will start to degrade and eventually become mechanically unstable. Thus, the water disintegrable catheter when in substantial contact with water in most instances will not support catheter functionality and will not support the hydrophilic coating. As the catheter breaks down the hydrophilic coating will become unstable and will not adhere to the catheter. Lab testing has confirmed this to be the case. It has also been shown that water-soluble outer layers such as polyvinylpyrrolidone (PVP) on top of PVOH will not overcome this problem even when cross-linked. In one embodiment, this disclosure provides a catheter that overcomes this problem by having a bi-layer catheter shaft tube that has a biodegradable outer layer (such as PLA) that will support the hydrophilic coating even when it's wetted. The outer layer bonds well to the water disintegrable inner layer but also allows a hydrophilic coating to be applied to the outer surface of the outer layer.

Fig. 1 illustrates portion of a catheter 10 according to the present disclosure. The catheter includes a catheter shaft that has a bi-layer tube 12 and a hydrophilic coating 14. In this embodiment, the tube 12 has a dual layer construction including a water disintegrable inner layer 16 and a biodegradable outer layer 18. The tube 12 may be made, for example, by coextrusion. A radial eyelet 20 extends through both layers 16, 18. A suitable tip (not shown) will be formed at the right end (as seen in Fig. 1) of the tube. Examples of suitable materials are PVOH for the inner layer 16 and PLA for the outer layer 18.

The biodegradable polymer outer layer 18 provides one or more of the following functionalities:
1) It acts as a primer for the hydrophilic coating 14.
2) It bonds well to the water disintegrable inner layer 16, which may be made of, for example, PVOH.
3) Initially, the bi-tube outer layer 18 acts as a water barrier when the hydrophilic coating 14 is hydrated, i.e. outer layer 18 stops or slows the water molecules from contacting the inner PVOH layer 16 and thus prevents the inner PVOH layer 16 from dissolving too quickly and causing the catheter coating 14 to fall off or break up. This is due to the fact that the outer layer 18 degrades mainly via hydrolysis and not purely on the basis of its water solubility (if it's a blend). Outer layer 18 is a water disintegrable material that dissolves at a slower rate than the inner layer 16. This in turn means it will protect the inner layer 16 from the wetted hydrophilic coating 14 (or water molecules) because it will remain impervious to water for the required duration.
4) The biodegradable outer layer 18 (e.g. PLA) is very thin. This means it will break down fast enough to meet flushability standards but will still protect the inner layer 16 long enough for full hydration of the hydrophilic coating 14 to occur and long enough for the patient to use the hydrophilically coated catheter 10. After the inner layer 16 dissolves first, a floppy outer skin will be left behind. It will flush easily and this skin is so thin (or is the correct blend of PLA/PVOH) so that it will break up into small enough pieces so that it will meet international standards for flushability. The PLA outer layer could be of low molecular weight so as to biodegrade very quickly and it could also have no cross linking so as to speed up the breakdown/dissolution of the outer layer once exposed to water. The PLA outer layer could also be blended with the likes of PVOH or PVP so as to facilitate a more rapid breakdown more suited to flushable products.
5) The outer layer 18 can be any suitable biodegradable polymer (e.g. PLA) or co-polymer or blend. Polyesters, polyglycolide, polyglycolic acid, poly lactic-co-glycolic acid, polylactide may also be suitable materials. The outer skin can be a blend of a biodegradable polymer and a water-soluble polymer (e.g. PVOH) or it can be exclusively biodegradable and not necessarily water-soluble. For example, PVOH is known to accelerate the degradation of PLA by increasing the hydrophilicity of the blend film and by breaking the crystallinity of PLA. Therefore, the hydrophilicity and degradability of PLA/PVA blend film can be controlled in a certain range by adjusting the proportion of PLA and PVA. Reference Pub Med.gov, US national Library of Med Institutes of Health, Sheng Wu Yi Xue Za Zhi 2008 Feb;25(1):139-42.

Internal testing has shown that PLA tubes (with very rough surfaces) manufactured via laser printing techniques, with a hydrophilic coating, achieve lubricity values comparable to a hydrophilic product. Accordingly, tubes with an outer layer of PLA can be coated with hydrophilic type coatings and can achieve desirable lubricity levels.

Tests have also shown that PVOH tubing has poor solubility in ethanol and methanol and that PVOH tubes can be successfully coated with hydrophilic coatings from the above solvents. However, when the hydrophilic coated PVOH tubes are wetted (with 100% water) the coating falls off because the water molecules dissolve the surface of the water-soluble PVOH tubing. As confirmed by the testing in the preceding paragraph, this will not happen to a PLA (or equivalent) overcoat on top of a PVOH layer.

Other surface features can be included to optimize lubricity. For example, grooves could be formed on the PLA layers. These could further aid lubricity by protecting the hydrophilic coating that is in between the grooves from abrasion.

In an example, a water flushable catheter includes a shaft made from a water disintegrable polymer wherein the hydrophilic coating is applied directly to the water disintegrable material. In this example, the hydrophilic coating is wetted/activated with a non-aqueous composition or a mixture of a non-aqueous composition and an amount of water. Such non-aqueous compositions may include PG, PEG, tetra-ethylene glycol, glycerol or tri-ethylene glycol or mixtures thereof. In one example, the mixture includes the non-aqueous composition and water wherein the amount of water is between about 0 wt% and about 20 wt% or between about 0 wt% and about 10 wt% or between about 0 wt% and about 5 wt% of the mixture. In one embodiment, the mixture includes PG and water. These non-aqueous compositions, alone or in a mixture with water, may also be used with a bi-layer tube as described above.

When non-aqueous compositions are used, alone or mixed with water, the catheter may be manufactured without the need for the customer to hydrate the coating immediately prior to use. That is, the hydrophilic coating may be activated during manufacturing or during packaging. This could be an advantage in terms of shelf life. It also retains the benefits of the product being "pre-hydrated" prior to the user opening the package and no manipulation would be required by the user to activate the coating. This may reduce the risk of spillage of the activation agent. Furthermore, packaged activated hydrophilic coating may be sterilized with ionizing radiation, such as gamma or e-beam radiation. For example, the packaged activated hydrophilic coating, which has been activated with a non-aqueous composition, may be sterilized with about 25kGy of gamma radiation.

The water disintegrable, flushable catheters disclosed herein provide the ability to utilize hydrophilic coatings on water disintegrable catheter shafts. Nobody would have expected one could have used such a coating on water disintegrable catheters because it would have been thought that the water used to activate the coating would have degraded the catheter to the point wherein the catheter could not be used. The water flushable catheters, however, provide a water disintegrable catheter that includes a hydrophilic or water based coating that achieves low CoF (coefficient of friction) values and can be flushed down the toilet for disposal thereof.

While the foregoing sets forth examples of suitable materials for making the flushable catheter of the present disclosure, it will be understood that other materials could be used. The catheter assembly may be made from one or more fluid (for example, water, urine or fluids utilized in toilet and plumbing systems) disintegrable materials. Such material may include, for example, water soluble, water hydrolysable or enzymatically hydrolysable materials, which dissolve or break down when in contact with water. The water disintegrable materials are preferably flushable materials which are suitable for disposal in a toilet or sanitary system and, even more preferably, biodegradable flushable materials which may be chemically broken down by water, living organisms or other biological means.

Such water disintegrable or enzymatically hydrolysable materials may include, for example, polyvinyl alcohol, including but not limited to an extrudable polyvinyl alcohol, polyacrylic acids, polylactic acid, polyesters, polyglycolide, polyglycolic acid, poly lactic-co-glycolic acid, polylactide, amines, polyacrylamides, poly(N-(2-Hydroxypropyl) methacrylamide), starch, modified starches or derivatives, amylopectin, pectin, xanthan, scleroglucan, dextrin, chitosans, chitins, agar, alginate, carrageenans, laminarin, saccharides, polysaccharides, sucrose, polyethylene oxide, polypropylene oxide, acrylics, polyacrylic acid blends, poly(methacrylic acid), polystyrene sulfonate, polyethylene sulfonate, lignin sulfonate, polymethacrylamides, copolymers of aminoalkyl-acrylamides and methacrylamides, melamine-formaldehyde copolymers, vinyl alcohol copolymers, cellulose ethers, poly-ethers, polyethylene oxide, blends of polyethylene-polypropylene glycol, carboxymethyl cellulose, guar gum, locust bean gum, hydroxyproply cellulose, vinylpyrrolidone polymers and copolymers, polyvinyl pyrrolidone-ethylene-vinyl acetate, polyvinyl pyrrolidone-carboxymethyl cellulose, carboxymethyl cellulose shellac, copolymers of vinylpyrrolidone with vinyl acetate, hydroxyethyl cellulose, gelatin, poly-caprolactone, poly(p-dioxanone), or combinations, blends or co-polymers of any of the above materials. The water disintegrable or enzymatically hydrolysable materials may also be any of those that are included in certified flushable products that meet the National Sanitation Foundation standards for flushability or materials and products that meet INDA/EDANA Flushability Guidelines or the UK Water Industry Research, test protocols set forth in "Test Protocol to Determine the Flushability of Disposable Products, Review of the Manufactures 3rd Ed. Guidance Document," 2013, by Drinkwater et al. While catheters made from water disintegrable or enzymatically hydrolysable materials may be disposed of in a toilet, it is not necessary to dispose of such catheters in a toilet and such catheters may also be disposed in normal municipal waste systems or garbage collection systems.

### Example I

CoFs of the coated and uncoated samples of PLA tubes, as an indicator of their lubricity, were measured using a Harland Friction Tester Model FTS5500. To determine the CoF of the tubes, a mandrel was inserted into 127 mm section of the coated or uncoated tube being tested. The tube was then clamped between two pieces of silicone rubber at 100g load wherein the silicone rubber had a Shore hardness of 60A. The tube with the mandrel inserted therein was pulled through the two pieces of silicone rubber at a speed of 10 mm/s. The force required to pull about 80 mm of the tube through the two pieces of silicone rubber was measured and recorded using a universal tensile tester equipped with a 200 N load cell. The CoF value was calculated from the ratio of recorded to applied loads (i.e., the recorded load divided by 2 times the applied load) when steady state was reached.

In a separate test, coated PLA tubes were abraded 25 times by passing the tubes through a hole which is just smaller than the outer diameter of the tubes. The hole was punched in a 1mm thick, silicone pad with Shore hardness of 60A. This test was designed to remove any portions of the coating that is not well adhered to the tubes. The CoFs of the abraded tubes were measured and an average CoF was calculated for each type of tube. Initial and abraded coefficient of friction (CoF) results, presented in the table below, are comparable to those for existing hydrophilic catheters.

**Coefficient of Friction Values**

| **Initial CoF** | **Abraded CoF (25 cycles)** | **Comment** |
|---|---|---|
| 0.0263 | 0.0263 | These are very rough uneven and curved tubes. Results are very likely to improve with extruded bilayer tubing |
| 0.0410 | 0.1204 | These are very rough uneven and curved tubes. Results are very likely to improve with extruded bilayer tubing |

| | | |
|---|---|---|
| Uncoated PLA control: 0.4248 (initial), 0.5478 (abraded) | | |

### Example II

### Tubes

A cross-head extrusion die was adapted to an injection moulding machine. Poly vinyl alcohol (PVOH) tubes were extruded at 195C. The extruded tubes were cooled with a pair of cooling inserts consisting of a split metal block mould with nominal diameter 5.2mm. The solid tubes were manually removed from the split mould after cooling.

### Coating:

PVOH tubes were dipped coated in a Primer UV curable coating solution and cured for approximately 30s. Primed PVOH tubes were dipped in a Top Coat UV polyvinyl pyrrolidone UV curable coating and subsequently cured for approximately 10 minutes. Coated catheters were immersed in propylene glycol (PG) for 5 minutes to lubricate the coating (activated catheters). Activated catheters were left standing vertically for 5 minutes to remove the excess PG liquid by gravity. Thus, the initial CoF values shown in the table below correspond to CoF after 5 minutes of dipping the catheters into PG activation agent

### Coefficient of friction (CoF):

CoF was measured using a Harland Friction Tester Model FTS5500. To determine the CoF of the tubes, a mandrel was inserted into 127 mm section of the coated or uncoated tube being tested. The tube was then clamped between two pieces of silicone rubber at 100g load wherein the silicone rubber had a Shore hardness of 60A. The tube with the mandrel inserted therein was pulled through the two pieces of silicone rubber at a speed of 10 mm/s. The force required to pull about 80 mm of the tube through the two pieces of silicone rubber was measured and recorded using a universal tensile tester equipped with a 200 N load cell. The CoF value was calculated from the ratio of recorded to applied loads (i.e., the recorded load divided by 2 times the applied load or 200g) when steady state was reached.

Coated tubes activated with PG were abraded 25 times by passing the tubes in air through a hoop 4.14mm in diameter (the relative diameters of the hoop and the tubes give an indication of the severity of the test). The hoop was punched in a 1mm thick, silicone pad with Shore hardness of 60A. This test was designed to remove any portions of the coating that is not well adhered to the tubes. The CoF of the abraded tubes were measured and an average CoF was calculated for each type of tube.

In a separate test, coated tubes activated with PG were left for an additional 10 minutes in the laboratory prior to testing the CoF. The table below shows a summary of 5 minutes, 15 minutes and abraded CoF (here 5 and 15 minutes is the time between the end of the dipping process in which PG is applied and the CoF test).

| **Sample no:** | **5 min CoF** | **Abraded CoF** | **15 min CoF** |
|---|---|---|---|
| 1 | 0.0512 | 0.0788 | 0.1117 |
| 2 | 0.1070 | 0.0959 | 0.1133 |
| 3 | 0.1035 | 0.0997 | 0.1026 |
| 4 | 0.1155 | 0.0930 | 0.0788 |
| **Average** | **0.0943** | **0.0918** | **0.1016** |

### Example III

*Tubes:* PVOH tubes were were manufactured by a traditional extrusion technique that used a water free cooling process..

*Coating:* PVOH tubes were dipped coated as in Example II. Coated catheters were immersed in 90% propylene glycol (PG) / 10% water for 5 minutes to activate the coating. Activated catheters were left standing vertically for 5 minutes to remove the excess PG/water by gravity. Thus, the initial CoF values shown in the table below correspond to CoF after 5 minutes of dipping the catheters into 90% PG/ 10% water activation agent

*Coefficient of friction (CoF):* CoF was measured using the same process as in Example II. Coated tubes activated with PG were abraded in air using the process of Example II. Then the tubes were abraded in water.

| **PVOH - Primer and Topcoat** | | | |
|---|---|---|---|
| **5mins Dwell in 90%PG / 10%Water** | | | |
| **Sample No.** | **Initial CoF** | **Abrasion In Air** | **Abrasion In Water** |
| **1** | 0.0461 | 0.1049 | n/a |
| **2** | 0.0475 | 0.0947 | n/a |
| **3** | 0.0441 | 0.1239 | 0.1425* |
| **Average:** | **0.0459** | **0.1078** | |
| **SD:** | **0.0017** | **0.0148** | |

| | | | |
|---|---|---|---|
| * Denotes that the sample had previously been abraded in air | | | |

### Example IV

*Tubes:* PVOH tubes were prepared in the same manner as in Example III.

*Coating:* PVOH tubes were dipped coated as in Example II. Coated catheters were force hydrated in water for 30 seconds to activate the coating. Force hydrate in the context of Examples IV, V and VI means to soak or dip or completely immerse the sample in water for 30 seconds prior to CoF testing.

*Coefficient of friction (CoF):* CoF was measured using the same process as in Example II. Coated tubes activated with PG were abraded in air using the process of Example II. MO in this table means maxed out and the average value was recorded

| **PVOH - Primer and Topcoat** | | |
|---|---|---|
| **30sec Force Hydrate in Water** | | |
| **Sample No.** | **Initial CoF** | **Abrasion In Air** |
| **1** | 0.783 | MO: 189.9 |
| **2** | 0.725 | MO: 189.8 |
| **3** | 0.7002 | MO: 170.9 |
| **Average:** | **0.7361** | **#N/A** |
| **SD:** | **0.0425** | **#N/A** |

### Example V

*Tubes:* Tubes were prepared having a single layer of PLA. The PLA tubes in examples V and VI are PLA only, i.e., the tubes are made from a single layer/piece of PLA manufactured on a laser printer. The PLA tubes are then subsequently dipped into the Primer and or Topcoat and indicated by the Table heading.

*Coating:* PLA tubes were dipped coated as in Example II. Coated catheters were force hydrated in water for 30 seconds to activate the coating.

*Coefficient of friction (CoF):* CoF was measured using the same process as in Example II. Coated tubes activated with PG were abraded in air using the process of Example II.

| **PLA - Primer and Topcoat** | | |
|---|---|---|
| **30sec Force Hydrate in Water** | | |
| **Sample No.** | **Initial CoF** | **Abrasion In Water** |
| **1** | 0.0066 | 0.0065 |
| **2** | 0.0142 | 0.0141 |
| **3** | 0.0126 | 0.1189 |
| **4** | 0.0068 | 0.0296 |
| **Average:** | **0.0101** | **0.0423** |
| **SD:** | **0.0039** | **0.0520** |

### Example VI

*Tubes:* Tubes were prepared having a single layer of PLA.

*Coating:* PLA tubes were dipped in a Top Coat UV polyvinyl pyrrolidone UV curable coating and subsequently cured for approximately 10 minutes. Coated catheters were force hydrated in water for 30 seconds to activate the coating.

*Coefficient of friction (CoF):* CoF was measured using the same process as in Example II. Coated tubes activated with PG were abraded in air using the process of Example II.

| **PLA - Topcoat only** | | |
|---|---|---|
| **30sec Force Hydrate in Water** | | |
| **Sample No.** | **Initial CoF** | **Abrasion In Water** |
| **1** | 0.0232 | 0.0526 |
| **2** | 0.0136 | 0.0105 |
| **3** | 0.0625 | 0.1596 |
| **4** | 0.1451 | 0.074 |
| **Average:** | **0.0611** | **0.0742** |
| **SD:** | **0.0599** | **0.0628** |

It should be understood that various changes and modifications to the presently preferred embodiments described herein will be apparent to those skilled in the art. Such changes and modification can be made without departing from the scope of the invention disclosed herein.

## Claims

1. A flushable urinary catheter comprising:
a bi-layer tube that has a water disintegrable inner layer and a biodegradable outer layer, wherein the outer layer dissolves at a slower rate than the inner layer; and
a hydrophilic coating on the outer layer.

2. The flushable urinary catheter of Claim 1 wherein the outer layer is made of polylactic acid.

3. The flushable urinary catheter of any one of the preceding claims wherein the inner layer is made of polyvinyl alcohol.

4. The flushable urinary catheter of any one of claims 1 - 3 wherein the outer layer is made of a material that degrades via hydrolysis.

5. The flushable urinary catheter of any one of claims 1 and 3 wherein the outer layer is made of a material that degrades via dissolution.

6. The flushable urinary catheter of any one of the preceding claims wherein the outer layer bonds well to the inner layer.

7. The flushable urinary catheter of any one of the preceding claims wherein the outer layer acts as a stable primer layer for the hydrophilic coating.

8. The flushable urinary catheter of any one of the preceding claims wherein the outer layer acts as a temporary water barrier that allows the hydrophilic coating to be hydrated but prevents water molecules attacking the inner layer.

9. The flushable urinary catheter of any one of the preceding claims further comprising at least one groove formed on the outer surface of the outer layer.

10. The flushable urinary catheter of any one of the preceding claims wherein the bi-layer tube comprises a co-extruded tube.

11. The flushable urinary catheter of any one of the preceding claims wherein the outer layer comprises a blend of polylactic acid and polyvinyl alcohol.

## Patentansprüche

1. Wegspülbarer Urinkatheter, umfassend:
eine zweischichtige Röhre, die eine im Wasser zerfallende Innenschicht und eine biologisch abbaubare Außenschicht aufweist, wobei sich die Außenschicht mit einer langsameren Geschwindigkeit auflöst als die Innenschicht; und
eine hydrophile Beschichtung auf der Außenschicht.

2. Wegspülbarer Urinkatheter nach Anspruch 1, wobei die Außenschicht aus Polylactid hergestellt ist.

3. Wegspülbarer Urinkatheter nach einem der vorstehenden Ansprüche, wobei die Innenschicht aus Polyvinylalkohol hergestellt ist.

4. Wegspülbarer Urinkatheter nach einem der Ansprüche 1 - 3, wobei die Außenschicht aus einem Material hergestellt ist, das sich über Hydrolyse zersetzt.

5. Wegspülbarer Urinkatheter nach einem der Ansprüche 1 und 3, wobei die Außenschicht aus einem Material hergestellt ist, das sich über Auflösung zersetzt.

6. Wegspülbarer Urinkatheter nach einem der vorstehenden Ansprüche, wobei sich die Außenschicht gut mit der Innenschicht verbindet.

7. Wegspülbarer Urinkatheter nach einem der vorstehenden Ansprüche, wobei die Außenschicht als eine stabile Primer-Schicht für die hydrophile Beschichtung fungiert.

8. Wegspülbarer Urinkatheter nach einem der vorstehenden Ansprüche, wobei die Außenschicht als eine vorübergehende Wassersperre fungiert, die es ermöglicht, dass die hydrophile Beschichtung hydriert wird, aber verhindert, dass Wassermoleküle die Innenschicht angreifen.

9. Wegspülbarer Urinkatheter nach einem der vorstehenden Ansprüche, ferner umfassend mindestens eine Nut, die an der Außenfläche der Außenschicht ausgebildet ist.

10. Wegspülbarer Urinkatheter nach einem der vorstehenden Ansprüche, wobei die zweischichtige Röhre eine koextrudierte Röhre umfasst.

11. Wegspülbarer Urinkatheter nach einem der vorstehenden Ansprüche, wobei die Außenschicht eine Mischung aus Polylactid und Polyvinylalkohol umfasst.

## Revendications

1. Cathéter urinaire à jeter dans les toilettes comprenant :
un tube bi-couche qui a une couche intérieure qui se désintègre dans l'eau et une couche extérieure biodégradable, dans lequel la couche extérieure se dissout plus lentement que la couche intérieure ; et
un revêtement hydrophile sur la couche extérieure.

2. Cathéter urinaire à jeter dans les toilettes selon la revendication 1 dans lequel la couche extérieure est en acide polylactique.

3. Cathéter urinaire à jeter dans les toilettes selon l'une quelconque des revendications précédentes dans lequel la couche intérieure est en alcool polyvinylique.

4. Cathéter urinaire à jeter dans les toilettes selon l'une quelconque des revendications 1 à 3 dans lequel la couche extérieure est en un matériau qui se dégrade par hydrolyse.

5. Cathéter urinaire à jeter dans les toilettes selon l'une quelconque des revendications 1 à 3 dans lequel la couche extérieure est en un matériau qui se dégrade par dissolution.

6. Cathéter urinaire à jeter dans les toilettes selon l'une quelconque des revendications précédentes dans lequel la couche extérieure se lie bien à la couche intérieure.

7. Cathéter urinaire à jeter dans les toilettes selon l'une quelconque des revendications précédentes dans lequel la couche extérieure agit comme une couche d'apprêt stable pour la couche de revêtement hydrophile.

8. Cathéter urinaire à jeter dans les toilettes selon l'une quelconque des revendications précédentes dans lequel la couche extérieure agit comme une barrière à l'eau temporaire qui permet que la couche hydrophile soit hydratée mais empêche les molécules d'eau d'attaquer la couche intérieure.

9. Cathéter urinaire à jeter dans les toilettes selon l'une quelconque des revendications précédentes comprenant en outre au moins un sillon formé sur la surface extérieure de la couche extérieure.

10. Cathéter urinaire à jeter dans les toilettes selon l'une quelconque des revendications précédentes dans lequel le tube bi-couche comprend un tube co-extrudé.

11. Cathéter urinaire à jeter dans les toilettes selon l'une quelconque des revendications précédentes dans lequel la couche extérieure comprend un mélange d'acide polylactique et d'alcool polyvinylique.
